# FASCICULE DE BREVET EUROPEEN

(11) **EP 3 322 386 B1**
(45) Date de publication et mention de la délivrance du brevet: **31.07.2024**
(21) Numéro de dépôt: 16741571.0
(22) Date de dépôt: 08.07.2016
(51) Int. Cl.: A61F 2/44

(54) **CAGE INTERVERTÉBRALE POUR ARTHRODÈSE**
BANDSCHEIBEN-CAGE FÜR ARTHRODESE
INTERVERTEBRAL CAGE FOR ARTHRODESIS

(30) Priorité: 16.07.2015 FR 1556698
(43) Date de publication de la demande: 23.05.2018
(73) Titulaire: Spineart SA, 1217 Meyrin (CH)
(72) Inventeur: LEVIEUX, Jérôme, 1242 Satigny (CH)
(74) Mandataire: Plasseraud IP
(86) Numéro de dépôt international: PCT/EP2016/066316
(87) Numéro de publication internationale: WO 2017/009242

(56) Documents cités:
- WO-A1-03/013396
- WO-A1-2014/018325
- DE-A1- 102008 044 951
- DE-U1- 202013 006 282
- FR-A1- 2 889 442
- US-A- 4 693 721
- US-A- 5 443 515
- US-A1- 2011 071 635
- US-A1- 2011 082 564
- US-A1- 2013 325 142
- US-A1- 2014 107 786

## Description

La présente invention concerne des cages intervertébrales pour arthrodèse, encore appelées « cages intersomatiques ».

L'arthrodèse est une opération chirurgicale qui consiste à fixer deux os initialement mobiles entre eux. Elle est pratiquée pour corriger une lésion dans une zone osseuse et faire disparaître les douleurs liées aux mouvements de cette lésion ; elle est irréversible. Elle est également nommée « fusion ».

Chez l'homme, la dégénérescence du disque intervertébral tend à réduire l'espace intradiscal et à rétrécir les foramens par lesquels les racines nerveuses sortent du canal rachidien. Cette dégénérescence fait partie des pathologies du rachis. Pour rappel, les disques intervertébraux se trouvent dans la colonne ertébrale entre deux vertèbres consécutives. Un disque intervertébral comprend un anneau de cartilage muni en son centre d'un noyau gélatineux. Les disques intervertébraux sont élastiques et contribuent ainsi à l'amortissement des chocs subis par une colonne vertébrale.

Le traitement chirurgical de la dégénérescence du disque intervertébral peut nécessiter l'arthrodèse (la fusion) d'un ou plusieurs segments vertébraux en recherchant la meilleure position anatomique possible. Cette technique est permet ainsi de bloquer définitivement une ou plusieurs des articulations intervertébrales de la colonne. Afin de restaurer l'espace normal et l'angle sagittal anatomique du segment (lordose), des cages intervertébrales sont implantées entre les vertèbres.

Les cages intervertébrales peuvent être pleines ou creuses (comme illustré dans les publications US 2011/082564 A1 et US 4 693 721 A).

Les cages intervertébrales creuses sont en général conçues comme une boite possédant deux orifices, un sur la face inférieure et un sur la face supérieure ; les faces latérales peuvent aussi être ajourées.

Le traitement chirurgical conduit en général à aviver les plateaux vertébraux et à employer un greffon osseux, présent au sein de la cage intervertébrale quand celle-ci est creuse et/ou autour de la cage, afin de favoriser la fusion des vertèbres.

On notera que dans la chirurgie du rachis lombaire, ces cages intervertébrales sont souvent installées par voie postérieure et qu'elles ont une forme plutôt allongée pour pouvoir passer entre les racines et le fourreau durai.

Dans ce qui suit, on entend par "hauteur" d'une cage la dimension
mesurée dans la direction correspondant à l'épaisseur (ou hauteur) d'un disque intervertébral.

De nombreuses études ont visé à favoriser la fusion des vertèbres en cherchant à offrir des moyens permettant d'améliorer la compatibilité et la relation des cages intervertébrales avec le tissu osseux; à titre d'exemple, on peut citer l'utilisation de reliefs et/ou de porosités sur la face inférieure et/ou supérieure qui peuvent être de nature à faciliter la fusion.

La compatibilité et la relation des cages intervertébrales avec le tissu osseux étant des paramètres critiques pour la qualité et la pérennité du traitement chirurgical, il existe un besoin toujours présent d'améliorer la relation entre une cage intervertébrale et le tissu osseux.

A cette fin, la présente invention propose une cage intervertébrale pour arthrodèse constituée :
- d'une face supérieure et une face inférieure comprenant respectivement une zone de contact supérieure et une zone de contact inférieure destinées à contacter respectivement une première vertèbre et une deuxième vertèbre consécutive à la première vertèbre, chacune de ces faces comprenant un orifice ;
- d'une partie reliant en continuité de matière la face supérieure et la face inférieure et configurée pour former une cavité destinée à recevoir un greffon osseux, où ladite cavité débouche sur les orifices de chacune des faces supérieure et inférieure ;
où au moins une partie de la zone de contact supérieure et/ou inférieure est constituée d'un matériau de titane poreux d'une épaisseur d'au moins 1 mm et d'une porosité comprise entre 50 % et 90 %, où le diamètre des pores est compris entre 200 µm et 1 mm et où les pores sont répartis de manière apériodique.

La présente invention concerne une cage telle que revendiquée ci-après. Les modes de réalisation préférés de l'invention sont décrits dans les revendications dépendantes.

On entend par « répartition des pores de manière apériodique » une répartition des pores selon une structure apériodique, c'est-à-dire selon une structure qui n'est pas construite sur la base d'un motif répété spatialement; on peut également qualifier une telle répartition d'« aléatoire » ou de « stochastique ».

Grâce à la présente invention, les inventeurs ont pu montrer qu'il est possible d'améliorer la compatibilité et la relation des cages intervertébrales avec le tissu osseux. On obtient ainsi des fusions stables et pérennes. On constate une bonne colonisation d'une telle cage intervertébrale par le tissu osseux, avec notamment une pénétration efficace de tissus osseux dans la partie de la zone de contact supérieure et/ou inférieure constituée d'un tel matériau de titane poreux. La relation entre une cage intervertébrale et le tissu osseux est ainsi avantageusement améliorée.

La présente invention vise également une cage intervertébrale pour arthrodèse comprenant les caractéristiques énoncées dans les modes de réalisations suivants, qui peuvent se combiner selon toutes les configurations techniquement envisageables :
- le matériau de titane poreux est constitué d'au moins 95 % en poids de titane métallique, par exemple d'au moins 99 % en poids de titane métallique ;
- la porosité du matériau de titane poreux est comprise entre 60 % et 8C) c>/O
- le diamètre des pores du matériau de titane poreux est compris entre 500 µm et 900 µm, par exemple entre 700 µm et 800 µm ;
- la partie de la zone de contact supérieure et/ou inférieure constituée d'un matériau de titane poreux occupe une surface supérieure ou égale à 30 % de la surface de la zone de contact respectivement supérieure et/ou inférieure ;
- le matériau de titane poreux est constitué d'une pluralité de filaments en matériau de titane dense, entrecroisés et en partie jointifs, où les pores sont formés par l'espace compris entre ces filaments et où les filaments sont de section sensiblement circulaire d'un diamètre compris entre 100 et 300 µm ;
- la partie reliant en continuité de matière la face supérieure et la face inférieure comprend une ouverture adaptée à recevoir un dispositif de préhension de ladite cage intervertébrale ;
- la cage intervertébrale est allongée, la longueur de la face supérieure et de la face inférieure est égale ou supérieure au double de leur largeur, par exemple la longueur est comprise entre 15 mm et 40 mm et la largeur est comprise entre 5 mm et 15 mm ;
- l'orifice de la face supérieure et celui de la face inférieure sont allongés et la longueur de chacun des orifices est égale ou supérieure au double de leur largeur, par exemple la longueur est comprise entre 10 mm et 20 mm et la largeur est comprise entre 3 mm et 8 mm ;
- le matériau de titane poreux, d'une porosité comprise entre 50 % et 90 %, où le diamètre des pores est compris entre 200 µm et 1 mm et où les pores sont répartis de manière apériodique, est produit par fabrication additive (aussi appelé impression en trois dimensions) ;
- la cage intervertébrale est constituée d'un matériau de titane de composition chimique sensiblement constante et la densité du matériau de titane diffère entre au moins deux parties de la cage; selon un autre mode de réalisation, la cage intervertébrale est constituée d'un ou plusieurs matériau(x) biocompatible(s) et du matériau de titane poreux, et la composition chimique du (ou desdits) matériau(x) biocompatible(s) diffère de celle du matériau de titane poreux.

D'autres particularités et avantages de la présente invention ressortiront dans la description ci-après d'exemples de réalisation non limitatifs, illustrés par les figures annexées, où :
Les figures 1 et 2 représentent des schémas de deux modes de réalisation de cages intervertébrales pour arthrodèse.
La figure 3 montre une image d'un exemple de matériau de titane poreux constituant au moins une partie de la zone de contact supérieure et/ou inférieure d'une cage intervertébrale pour arthrodèse selon la présente invention.

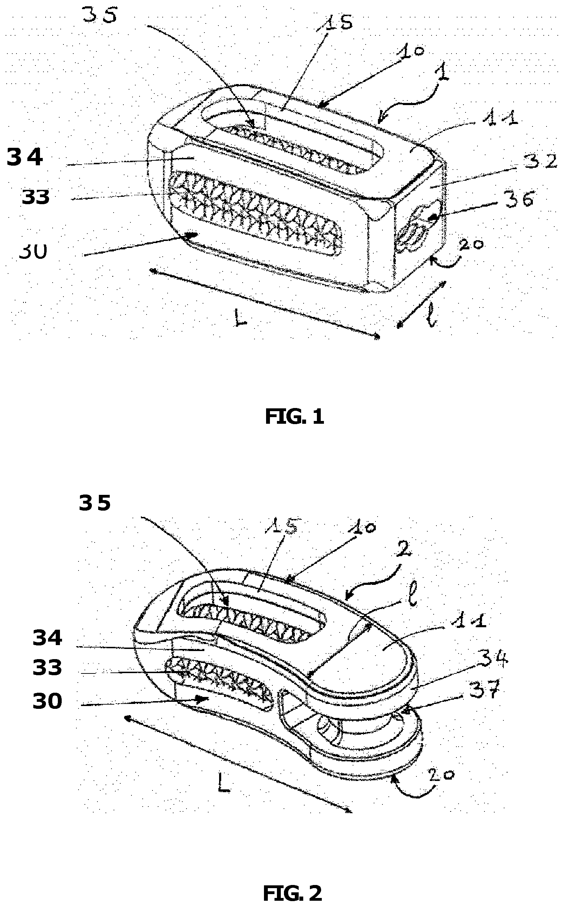

Les cages intervertébrales pour arthrodèse représentées sur les figures 1 et 2, respectivement notées 1 et 2, comprennent une face supérieure 10 et une face inférieure 20 ; chacune de ces faces comprend une zone de contact, respectivement supérieure 11 et inférieure (non représentée), destinée à contacter respectivement une première vertèbre et une deuxième vertèbre consécutive à la première vertèbre ; chacune de ces faces comprend également un orifice 15 ; dans les cages représentées, la face supérieure 10 est la symétrie miroir de la face inférieure 20. Les deux faces, supérieure et inférieure, sont en regard l'une de l'autre et sont en général légèrement inclinées l'une par rapport à l'autre, d'un angle par exemple compris entre 0° et 20° ; cet angle correspond à la lordose entre les deux vertèbres consécutives. Ces deux faces sont reliées en continuité de matière par une partie 30. Cette partie 30 comprend notamment une paroi qui est en général sensiblement perpendiculaire aux faces supérieure et inférieure. Cette paroi peut faire le tour du pourtour des faces supérieure et inférieure comme représenté en figure 1 ou seulement d'une partie du pourtour des faces supérieure et inférieure comme représenté en figure 2. Cette partie 30 assure la rigidité mécanique de la cage intervertébrale et est configurée pour permettre de résister aux efforts appliqués entre deux vertèbres consécutives. La partie 30 est configurée pour former une cavité 35 destinée à recevoir un greffon osseux. Ladite cavité 35 débouche sur les orifices de chacune des faces supérieure et inférieure. De telles cages intervertébrales sont dites « monoblocs » et la face supérieure 10 n'est pas mobile par rapport à la face inférieure 20.

Les cages intervertébrales pour arthrodèse des figures 1 et 2 sont allongées et la longueur L de la face supérieure (dans ce cas similaire à celle de la face inférieure) est égale ou supérieure au double de leur largeur l ; à titre d'exemple cette longueur est comprise entre 15 mm et 40 mm et cette largeur est comprise entre 5 mm et 15 mm.

La partie 30 reliant en continuité de matière les deux faces supérieure et inférieure comprend deux faces longitudinales 31 disposées selon un axe correspondant à la longueur et deux faces latérales 32 (pour la figure 1) ou 34 (pour la figure 2) disposées selon un axe correspondant à la largeur. Dans les exemples représentés, les faces longitudinales 31 comprennent des zones 33 amincies, par exemple formées par une structure en résille. Ces zones peuvent être partiellement ou totalement dépourvues de matière.

Les cages intervertébrales pour arthrodèse des figures 1 et 2 ont un orifice sur la face supérieure (similaire à celui de la face inférieure) allongé et la longueur de l'orifice est égale ou supérieure au double de sa largeur ; par exemple cette longueur est comprise entre 10 mm et 25 mm et cette largeur est comprise entre 3 mm et 8 mm.

La cage intervertébrale pour arthrodèse 1 de la figure 1 est sensiblement parallélépipédique et comprend deux faces longitudinales parallèles et deux faces latérales parallèles. Ces faces longitudinales et latérales sont perpendiculaires entre elles et en général légèrement inclinées par rapport à la normale de la face supérieure et/ou par rapport à la normale de la face inférieure de ladite cage. Une face latérale comprend par ailleurs une cavité 36; cette cavité est destinée à recevoir une extrémité d'une pièce faisant partie d'un dispositif de préhension de la cage intervertébrale ; un tel dispositif de préhension est de nature à permettre au chirurgien de manipuler puis de disposer la cage intervertébrale entre deux vertèbres. Dans le cas du présent exemple, la cavité disposée sur une des faces latérales est filetée et permet à la pièce faisant partie d'un dispositif de préhension de s'y fixer, cette pièce est du type d'une vis.

La cage intervertébrale pour arthrodèse 2 de la figure 2 est de forme en « croissant » et comprend deux faces longitudinales incurvées, parallèles l'une à l'autre. Les deux faces latérales sont sensiblement de forme demi-cylindrique, chacune en symétrie miroir de l'autre, et rejoignent en les tangeantant les faces longitudinales incurvées. Ces faces longitudinales et latérales sont en général légèrement inclinées par rapport à la normale de la face supérieure et/ou par rapport à la normale de la face inférieure de ladite cage. Une face latérale comprend par ailleurs une zone 37 qui est destinée à coopérer avec une extrémité d'une pièce faisant partie d'un dispositif de préhension de la cage intervertébrale. Dans le cas du présent exemple, ladite zone disposée sur une des faces latérales comprend une partie cylindrique située entre la face supérieure et la face inférieure de la cage et en retrait des pourtours de ces faces. Cette partie cylindrique permet de manipuler la cage grâce à un dispositif de préhension comprenant une partie du type d'une pince.

Dans les présents exemples selon l'invention, les zones de contact, supérieure et inférieure, des faces respectivement supérieure et inférieure des cages intervertébrales représentées sur les figures 1 et 2 sont constituées d'un matériau de titane poreux d'une épaisseur d'au moins 1 mm et d'une porosité comprise entre 50 % et 90 %, où le diamètre des pores, DP, est compris entre 200 µm et 1 mm et où les pores sont répartis de manière apériodique.

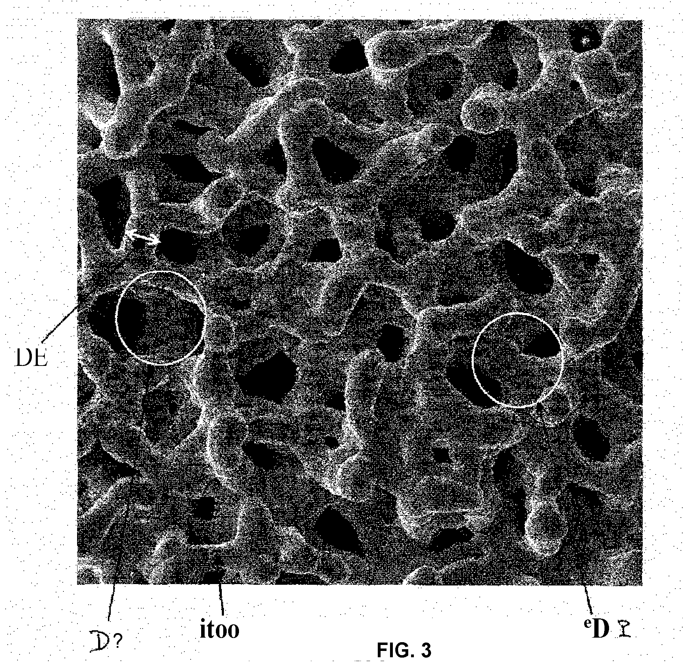

La figure 3 montre une image d'un exemple d'un tel matériau de titane poreux. Cette image a été prise grâce à un microscope électronique à balayage dont la tension du faisceau d'électrons est réglée à 15 kV. L'image est un carré dont la dimension d'un côté correspond à une dimension de 3,6 mm. On voit distinctement apparaitre un matériau continu constitué d'une pluralité de filaments 100 se rejoignant par endroits et séparés par du vide en autres endroits. Le matériau est donc de type « ramifié » selon une structure tridimensionnelle. Les filaments, entrecroisés et en partie jointifs, sont en matériau de titane dense. L'espace compris entre ces filaments forme une pluralité de pores. Dans l'exemple représenté, les filaments sont de section sensiblement circulaire d'un diamètre, DF, sensiblement égal à 200 µm et les pores ont un diamètre, DP, sensiblement compris entre 500 µm et 700 µm. Les pores sont répartis de manière apériodique.

Selon un mode de réalisation, le matériau de titane poreux est constitué de titane non allié (contenant au moins 99% de titane en poids) ; selon un autre mode de réalisation, le matériau de titane poreux est constitué de titane allié, par exemple d'un alliage comprenant de l'aluminium et du vanadium, connu sous la nomenclature Ti6Al4V ou TA6V. De tels matériaux sont biocompatibles.

Selon un mode de réalisation, l'ensemble de la cage intervertébrale est composé d'un unique matériau de titane et seule la densité de ce matériau varie en fonction des parties de la cage intervertébrale; selon un autre mode de réalisation, la cage intervertébrale est constituée d'un ou plusieurs matériau(x) biocompatible(s) et du matériau de titane poreux et la composition chimique du (ou desdits) matériau(x) biocompatible(s) diffère de celle du matériau de titane poreux. A titre d'exemple, le matériau biocompatible, différent du matériau de titane poreux, est un polymère, par exemple connu sous la dénomination de PEEK. Dans un tel exemple, il est possible de réaliser l'essentiel de la cage intervertébrale dans ledit matériau biocompatible et d'adjoindre sur une face supérieure et/ou une face inférieure de la cage intervertébrale au moins une partie de la zone de contact constituée d'un matériau de titane poreux. Une telle adjonction peut être réalisée par toute technique connue d'un homme du métier susceptible de permettre de « rapporter » un matériau de titane poreux sur un autre matériau.

Selon un mode de réalisation, le matériau de titane poreux est fabriqué par fabrication additive (autrement appelé impression en trois dimensions). On entend par « fabrication additive » des procédés de fabrications d'ajout de matière, la plupart du temps assistés par ordinateur. Elle est définie par l'ASTM comme étant le procédé de mise en forme d'une pièce par ajout de matière, par empilement de couches successives, en opposition aux procédés par retrait de matière, tel que l'usinage. On donne communément le nom d' « impression tridimensionnelle » (impression 3D) à une telle technologie. On cite à titre d'exemple des procédés tels que le frittage par laser, comme notamment le frittage sélectif laser (SLS), le frittage laser direct de métal (DMLS) ou encore le E-Beam (EBM). Des « imprimantes » utilisent un laser qui vient durcir une poudre métallique en certains endroits, pour donner forme à l'objet final. On cite également à titre d'exemple des procédés tels que le frittage par faisceaux d'électrons, connu notamment sous l'appellation « EBM » pour « Electron Beam Melting ».

## Revendications

1. Cage intervertébrale (1, 2) pour arthrodèse constituée :
-(a)- d'une face supérieure (10) et une face inférieure (20) comprenant respectivement une zone de contact supérieure (11) et une zone de contact inférieure destinées à contacter respectivement une première vertèbre et une deuxième vertèbre consécutive à la première vertèbre, chacune de ces faces comprenant un orifice (15) ;
-(b)- d'une partie (30) reliant en continuité de matière la face supérieure (10) et la face inférieure (20) et configurée pour former une cavité (35) destinée à recevoir un greffon osseux, où ladite cavité débouche sur les orifices de chacune des faces supérieure et inférieure ;
**caractérisée en ce que**
-(c)- au moins une partie de la zone de contact supérieure et/ou inférieure est constituée d'un matériau de titane poreux d'une épaisseur d'au moins 1 mm et d'une porosité comprise entre 50 % et 90 %,
-(d)- le diamètre des pores, DP, est compris entre 200 µm et 1 mm
-(e)- les pores sont répartis de manière apériodique
(f)⁻le matériau de titane poreux est un matériau continu constitué d'une pluralité de filaments (100) en matériau de titane dense, entrecroisés, en partie jointifs, se rejoignant par endroits et séparés par du vide en autres endroits;
-(f)- les pores sont formés par l'espace compris entre les filaments;
-(g)- Le matériau est de type « ramifié » selon une structure tridimensionnelle
-(h)- les filaments sont de section sensiblement circulaire d'un diamètre, DF, compris entre 100 et 300 µm;
-(i)- le matériau de titane poreux est produit par fabrication additive;
-(j)- le matériau de titane est de composition chimique sensiblement constante;
-(k)- la densité du matériau de titane diffère entre au moins deux parties de la cage.

2. Cage intervertébrale selon l'une des revendications précédentes **caractérisée en ce que** la porosité du matériau de titane poreux est comprise entre 60 % et 80 %.

3. Cage intervertébrale selon l'une des revendications précédentes **caractérisée en ce que** le diamètre des pores du matériau de titane poreux est compris entre 500 µm et 900 µm, par exemple entre 700 µm et 800 µm.

4. Cage intervertébrale selon l'une des revendications précédentes **caractérisée en ce que** la partie de la zone de contact supérieure et/ou inférieure constituée d'un matériau de titane poreux occupe une surface supérieure ou égale à 30 % de la surface de la zone de contact respectivement supérieure et/ou inférieure.

5. Cage intervertébrale selon l'une des revendications précédentes **caractérisée en ce que** la partie reliant en continuité de matière la face supérieure et la face inférieure comprend une ouverture (36, 37) adaptée à recevoir un dispositif de préhension de ladite cage intervertébrale.

6. Cage intervertébrale selon l'une des revendications précédentes **caractérisée en ce que** la cage intervertébrale est allongée et **en ce que** la longueur de la face supérieure et de la face inférieure est égale ou supérieure au double de leur largeur, par exemple d'une longueur comprise entre 15 mm et 40 mm et d'une largeur comprise entre 5 mm et 15 mm.

7. Cage intervertébrale selon la revendication précédente **caractérisée en ce que** l'orifice de la face supérieure et celui de la face inférieure sont allongés et **en ce que** la longueur de chacun des orifices est égale ou supérieure au double de leur largeur, par exemple d'une longueur comprise entre 10 mm et 25 mm et d'une largeur comprise entre 3 mm et 8 mm.

## Patentansprüche

1. Intervertebraler Cage (1, 2) für die Arthrodese, umfassend:
a) eine obere Fläche (10) und eine untere Fläche (20), die jeweils einen oberen Kontaktbereich (11) und einen unteren Kontaktbereich aufweisen, dazu bestimmt, mit einem ersten Wirbel bzw. mit einem dem ersten Wirbel folgenden zweiten Wirbel, in Kontakt zu kommen, wobei jede dieser Flächen eine Öffnung (15) aufweist,
b) einen Teil (30), der die obere Fläche (10) und die untere Fläche (20) materialschlüssig verbindet und dazu ausgebildet ist, einen Hohlraum (35) zu bilden, der dazu bestimmt ist, ein Knochentransplantat aufzunehmen, wobei der Hohlraum in die Öffnungen der oberen und der unteren Fläche mündet, **dadurch gekennzeichnet, dass**
c) wenigstens ein Teil des oberen und/oder unteren Kontaktbereichs aus einem porösen Titanmaterial mit einer Dicke von wenigstens 1 mm und einer Porosität zwischen 50 % und 90 % besteht,
d) der Porendurchmesser DP zwischen 200 µm und 1 mm liegt,
e) die Poren aperiodisch verteilt sind, f) das poröse Titanmaterial ein kontinuierliches Material ist, das aus einer Vielzahl von Filamenten (100) aus dichtem Titanmaterial besteht, die sich kreuzen, teilweise aneinander grenzen, an einigen Stellen zusammenlaufen und an anderen Stellen durch Hohlräume getrennt sind,
f) die Poren durch den Raum zwischen den Filamenten gebildet werden,
g) das Material vom Typ "verzweigt" ist, mit einer dreidimensionalen Struktur,
h) die Filamente einen im wesentlichen kreisförmigen Querschnitt mit einem Durchmesser DF zwischen 100 und 300 µm aufweisen,
i) das poröse Titanmaterial durch additive Fertigung hergestellt ist;
j) das Titanmaterial eine im wesentlichen konstante chemische Zusammensetzung aufweist;
k) sich die Dichte des Titanmaterials bei wenigstens zwei Teilen des Käfigs unterscheidet.

2. Intervertebraler Cage nach einem der vorhergehenden Ansprüche, **dadurch gekennzeichnet, dass** die Porosität des porösen Titanmaterials zwischen 60 % und 80 % liegt.

3. Intervertebraler Cage nach einem der vorhergehenden Ansprüche, **dadurch gekennzeichnet, dass** der Porendurchmesser des porösen Titanmaterials zwischen 500 µm und 900 µm, beispielsweise zwischen 700 µm und 800 µm, liegt.

4. Intervertebraler Cage nach einem der vorhergehenden Ansprüche, **dadurch gekennzeichnet, dass** derTeil des oberen und/oder unteren Kontaktbereichs, der aus porösem Titanmaterial besteht, eine Fläche einnimmt, die größer oder gleich 30 % der Fläche des jeweiligen oberen und/oder unteren Kontaktbereichs ist.

5. Intervertebraler Cage nach einem der vorhergehenden Ansprüche, **dadurch gekennzeichnet, dass** der die Oberseite und die Unterseite materialschlüssig verbindende Teil eine Öffnung (36, 37) aufweist, die dazu ausgebildet ist, eine Vorrichtung zum Greifen des intervertebralen Cages aufzunehmen.

6. Intervertebraler Cage nach einem der vorhergehenden Ansprüche, **dadurch gekennzeichnet, dass** der intervertebraler Cage länglich ist und dass die Länge der Oberseite und der Unterseite gleich oder größer als das Doppelte ihrer Breite ist, beispielsweise mit einer Länge zwischen 15 mm und 40 mm und einer Breite zwischen 5 mm und 15 mm.

7. Intervertebraler Cage nach dem vorhergehenden Anspruch, **dadurch gekennzeichnet, dass** die Öffnung der Oberseite und die Öffnung der Unterseite länglich sind und dass die Länge jeder der Öffnungen gleich oder größer als das Doppelte ihrer Breite ist, beispielsweise mit einer Länge zwischen 10 mm und 25 mm und einer Breite zwischen 3 mm und 8 mm.

## Claims

1. An intervertebral cage (1, 2) for arthrodesis, composed of:
(a) an upper face (10) and a lower face (20) comprising, respectively, an upper contact zone (11) and a lower contact zone which are intended to contact, respectively, a first vertebra and a second vertebra consecutive to the first vertebra, each of these faces comprising an orifice (15);
(b) a part (30) connecting the upper face (10) and the lower face (20), with continuity of material, and configured to form a cavity (35) intended to receive a bone graft, where said cavity opens out at the orifices of each of the upper and lower faces;
**characterized in that**
(c) at least part of the upper and/or lower contact zone is made of a porous titanium material with a thickness of at least 1 mm and with a porosity of between 50% and 90%;
(d) the diameter of the pores (DP) is between 200 µm and 1 mm;
(e) the pores have an aperiodic distribution;
(f) the porous titanium material is a continuous material composed of a plurality of filaments of dense titanium material which are interlaced and in part join each other that join each other at some places and are separated by an empty space at other places;
(g) the pores are formed by the space between these filaments (100);
(h) the material is a "branched" type material, according to a three-dimensional structure;
(i) the filaments are of a substantially circular cross section with a diameter DF of between 100 and 300 µm;
(j) the porous titanium material is produced by additive manufacturing;
(k) the titanium material is of substantially constant chemical composition;
(l) the density of the titanium material differs between at least two parts of the cage.

2. The intervertebral cage as claimed in claim 1, **characterized in that** the porosity of the porous titanium material is between 60% and 80%.

3. The intervertebral cage as claimed in one of the preceding claims, **characterized in that** the diameter of the pores of the porous titanium material is between 500 µm and 900 µm, for example between 700 µm and 800 µm.

4. The intervertebral cage as claimed in one of the preceding claims, **characterized in that** the part of the upper and/or lower contact zone made of a porous titanium material occupies a surface area of greater than or equal to 30% of the surface area of the upper and/or lower contact zone, respectively.

5. The intervertebral cage as claimed in one of the preceding claims, **characterized in that** the part connecting with upper face to the lower face, with continuity of material, comprises an opening (36, 37) suitable for receiving a device for gripping said intervertebral cage.

6. The intervertebral cage as claimed in one of the preceding claims, **characterized in that** the intervertebral cage is elongate, and **in that** the length of the upper face and of the lower face is equal to or greater than twice their width, for example with a length of between 15 mm and 40 mm and a width of between 5 mm and 15 mm.

7. The intervertebral cage as claimed in the preceding claim, **characterized in that** the orifice of the upper face and the orifice of the lower face are elongate, and **in that** the length of each of the orifices is equal to or greater than twice their width, for example with a length of between 10 mm and 25 mm and a width of between 3 mm and 8 mm.
